# NEW EUROPEAN PATENT SPECIFICATION

(11) **EP 2 231 118 B2**
(45) Date of publication and mention of the opposition decision: **17.05.2017**
(45) Mention of the grant of the patent: 04.12.2013
(21) Application number: 08861465.6
(22) Date of filing: 17.12.2008
(51) Int. Cl.: A61K 9/10, A61K 31/397

(54) **SUSPENSION COMPRISING NON-MICRONIZED EZETIMIBE MICRO-PARTICLES**
SUSPENSION MIT NICHT-MIKRONISIERTEN MIKROPARTIKELN VON EZETIMIB
SUSPENSION COMPRENANT DES MICROPARTICULES D'ÉZÉTIMIBE NON MICRONISÉES

(30) Priority: 17.12.2007 SI 200700331; 24.01.2008 SI 200800017
(43) Date of publication of application: 29.09.2010
(73) Proprietor: KRKA, tovarna zdravil, d.d., Novo mesto, 8501 Novo mesto (SI)
(72) Inventor: BENKIC, Primoz, 1000 Ljubljana (SI); SMRKOLJ, Matej, 1411 Izlake Zagorje Ob Savi (SI); KLJAJIC, Alen, 3000 Celje (SI); SEDMAK, Gregor, 1000 Ljubljana (SI)
(74) Representative: Uexküll & Stolberg
(86) International application number: PCT/EP2008/067808
(87) International publication number: WO 2009/077573

(56) References cited:
- WO-A1-2006/060808
- WO-A1-2007/003365

## Description

### FIELD OF THE INVENTION

The present invention relates to an efficient and simple process for the preparation of a suspension comprising non-micronized ezetimibe micro-particles having small particle size and high specific surface area wherein said ezetimibe micro-particles are prepared without micronization process and wherein said process prevents secondary agglomeration of ezetimibe micro-particles in the suspension.

### BACKGROUND OF THE INVENTION

Ezetimibe with chemical name 1-(4-fluorophenyl)-3(R)-[3-(4-fluorophenyl)-3(S)-hydroxypropyl]-4(S)-(4-hydroxyphenyl)-2-azetidinone and structural formula (I) 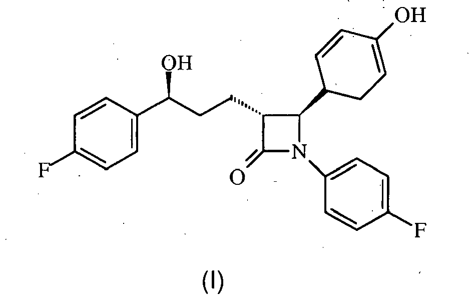 is representative of a class of compounds known as lipid-lowering compounds that selectively inhibits the intestinal absorption of cholesterol and related phytosterols. Its mechanism of action differs from those of other classes of cholesterol lowering compounds, such as HMG-CoA reductase inhibitors. It does not inhibit cholesterol synthesis in the liver or increase bile excretion but inhibits absorption of cholesterol, leading to a decrease in the delivery of intestinal cholesterol to the liver. Such mechanism is complementary to that of HMG-CoA reductase inhibitors. Ezetimibe is marketed under the tradename ZETIA^{®} or EZETROL^{®}, which is available as a tablet for oral administration.

Ezetimibe substance, its synthesis pathways and its use for the treatment or prevention of atherosclerosis, or for the reduction of plasma cholesterol levels was first disclosed in EP 0 720 599 B1. Different synthetic routes to ezetimibe and derivatives thereof have been described in literature, as for example in US 5,856,473, EP 0 906 278 B1, EP 1 169 468 A1 and in EP 1 137 634 B1.

Polymorphic forms of ezetimibe are described for example in WO 2005/009955, WO 2005/062897, WO 2006/060808, US 2006/0234996, IPCOM000131677D disclosing mainly anhydrous and hydrous crystalline forms, different mixtures thereof and amorphous form of ezetimibe. The obtained polymorphic form depends on the solvent used in the recrystallization step and on the water content of the final product (WO 2006/060808). Ezetimibe form A, form B and the process for the preparation thereof is disclosed in WO 2006/060808. The solvated forms of ezetimibe form B are disclosed in IPCOM000131677D and ezetimibe tert-butanol solvate is disclosed in co-pending patent application EP 07 001 537.5.

Ezetimibe is included, in several different formulations, described as for example in EP 1 353 696 B1, which discloses a composition comprising ezetimibe, lactose monohydrate, microcrystalline cellulose, povidone, croscarmellose sodium, sodium lauryl sulfate and magnesium stearate, WO 03/055464, which discloses a micronized pharmaceutical powder with immediate release having a grain size distribution of not more than 100 µm, and comprising of at least an active substance, at least a wetting agent and at least a diluent, and WO 03/068186, which discloses formulation, comprising active ingredients of a great variety and a pharmaceutically acceptable vehicle comprising at least one compound selected from the group consisting of hydrophilic surfactant, a lipophilic surfactant, a triglyceride and a solubilizer.

Ezetimibe is practically insoluble in water, which causes it to exhibit a low dissolution rate in aqueous media, e.g. gastrointestinal fluids, which can result in low bioavailability after oral ingestion. WO 2006/060808 solves the low-solubility problem of ezetimibe by preparing micronized ezetimibe having much higher specific surface area than the non-micronized form. Micronized ezetimibe particles have a particle size of less than about 30 microns. However, the micronization process is limited by the particle size and by poor bulk flow and handling. WO2007011349 discloses the method of achieving higher specific surface area by preparing a granulate comprising an active pharmaceutical ingredient having a poor water solubility intimately associated with at least one pharmaceutical acceptable sugar. WO 2007073389 discloses the process for the preparation of a pharmaceutical composition having low aqueous solubility by fixing the drug in a strong matrix comprising at least one at least partially amorphous sugar to obtain sugar-matrix and milling the sugar-drug matrix to obtain a milled sugar-drug matrix as a pharmaceutical composition. WO 2007/103453 solves the problem of low solubility of ezetimibe by the addition of a hydrophilic excipient, such as a saccharide or polysaccharide to a composition containing milled ezetimibe.

US 2007/0275052 is directed to a pharmaceutical composition comprising micronized ezetimibe of which about 90% of the particles are not more than about 25 microns and of which about 50% of the particles are not more than about 4 microns. The application is silent how said small micronized particles of ezetimibe are prepared and how said small micronized particles are incorporated in the pharmaceutical composition.

As shown above the particle size reduction and concomitant increase in surface area of a low soluble active substance is not always effective enough for increasing the dissolution rate and bioavailability of the active substance due to limitation in particle size and corresponding negative bulk properties of micronized powder during the formulation process. It is known that many low soluble drugs show a strong triboelectric effects linked to chargeability of small particles with high specific surface area and a strong tendency to agglomeration. Consequences of negative bulk properties of micronized material are reduced flow characteristics of bulk material, moreover, when the drug is micronized to very fine particles, agglomeration can easily occur, particularly during the process of the preparation of the pharmaceutical composition. This can significantly reduce the specific surface area of micronized material incorporated into pharmaceutical composition, which adequately lowers the bioavailability of the low soluble active substance.

When using micronized material comprising fine particles it is very difficult to assure appropriate content and content uniformity of the active ingredient due to loss of particles during the manufacturing procedure while exposing particles to exhaust air that exits the fluid-bed apparatus that is a standard equipment in fluid-bed granulation process. Another drawback of micronisation is the possibility of amorphisation of an active substance particles surface. It is known that the amorphous form is chemically and physically unstable and it can transform into crystalline form. Larger crystalline particles with lower dissolution rate often form during transformation. Higher formation of degradation products can also be observed during pharmaceutical processes and during the storage of final drug product containing such surface amorphisized particles of active substance.

Therefore, there would be a significant contribution to the art to provide a suspension comprising ezetimibe particles that show high bioavailability with improved dissolution rate and that could be easily and directly used in the process of the preparation of pharmaceutical composition comprising ezetimibe.

The above mentioned problems have been solved by preparing a suspension comprising non-micronized ezetimibe micro-particles having small particle size and high specific surface area wherein said ezetimibe micro-particles are prepared without micronization process, and wherein said process prevents secondary agglomeration of said ezetimibe micro-particles in the suspension and wherein said suspension of non-micronized ezetimibe micro-particles can be directly used in the process of the preparation of the pharmaceutical composition.

### SUMMARY OF THE INVENTION

The first embodiment of the present invention relates to a process of the preparation of a suspension comprising non-micronized ezetimibe micro-particles having small particle size and high specific surface area comprising the steps of (i) suspending ezetimibe particles in a solvent and (ii) de-agglomeration and homogenization the suspension by high shear mixer or colloid mill or generally a wet homogenizer.

The second embodiment of the present invention relates to process of the preparation of a suspension comprising non-micronized ezetimibe micro-particles having small particle size and high specific surface area comprising the steps of (i) suspending ezetimibe tert-butanol solvate in a solvent, preferably in water and (ii) de-agglomerating and homogenizing of the obtained suspension of ezetimibe by a wet homogenizer preferably a high pressure homogenizer and in particular a high shear mixer or colloid mill.

The third embodiment of the present invention relates to a process of the preparation of a suspension comprising non-micronized ezetimibe micro-particles having small particle size and high specific surface area from needle shaped particles of average size of more than 30 µm by (i) suspending the needle shaped ezetimibe particles in a solvent, preferably in water and (ii) de-agglomerating/milling and homogenizing of the obtained suspension of ezetimibe by wet homogenizer. Preferably the particles have a size of less than 100 µm.

Step (i) is in each case conducted by (i-a) dissolving ezetimibe in a solvent, (i-b) adding the solution of step (i-a) to an anti-solvent, (i-c) recovering the obtained precipitate from the resulting suspension, (i-d) drying the precipitate, (i-e) suspending the dried precipitate in a solvent.

The suspension comprises non-micronized ezetimibe micro-particles having small particle size and high specific surface area wherein non-micronized ezetimibe micro-particles have primary particle size of less than 30 µm, preferably less than 20 µm, more preferably less than 10 µm and most preferably less than 5 µm, and specific surface area of at least 2.5 m²/g, preferably at least 3 m²/g. More preferably the surface area is between 2.5 m²/g and 10 m²/g and most preferably between 3 and 10 m²/g. The particles preferably have a size or more than 0.5 µm and more preferably of more than 1 µm.

Another embodiment of the present invention relates to a process for preparing a pharmaceutical composition comprising non-micronized ezetimibe micro-particles and optionally one or more other active ingredient and at least one pharmaceutical acceptable excipient.

The pharmaceutical composition comprising non-micronized ezetimibe micro-particles and optionally one or more other active ingredient and at least one pharmaceutical acceptable excipient can be used in the treatment of lipid disorders.

### BRIEF DESCRIPTION OF THE DRAWINGS

Figure 1: Morphology of ezetimibe particles prepared according to Example 1
Figure 2: Morphology of ezetimibe particles prepared according to Example 2
Figure 3: Morphology of agglomerated ezetimibe micro-particles prepared according to Example 4
Figure 4: Bimodal particle size distribution of agglomerates of Example 4a.
Figure 4a: Morphology of ezetimibe particles prepared according to Example 4b.
Figure 5: Particle size distribution of the suspension comprising non-micronized ezetimibe micro-particles having small particle size and high specific surface area prepared according to Example 5a.
Figure 5a: Particle size distribution of the suspension comprising ezetimibe micro-particles having small particle size and high specific surface area prepared according to Example 5c.
Figure 6: Morphology of agglomerated ezetimibe micro-particles prepared according to Example 6
Figure 7: Bimodal particle size distribution of agglomerates of Example 6.
Figure 8: Particle size distribution of the suspension comprising ezetimibe micro-particles having small particle size and high specific surface area prepared according to Example 7.
Figure 9: Morphology of non-micronized ezetimibe micro-particles of small particle size and high specific surface area after recovering from suspension, showing needle-shaped primary particles.
Figure 10: Particle size distribution of the suspension comprising ezetimibe micro-particles having small particle size and high specific surface area prepared according to Example 8.

### DETAILED DESCRIPTION OF THE INVENTION

The inventors of the present invention surprisingly found out that besides small particle size and high specific surface area of ezetimibe particles the secondary agglomeration thereof during process of the preparation of the pharmaceutical composition is a major drawback in providing required bioavailable product. Moreover, the inventors found that when reducing the particle size of ezetimibe by micronization processes the chargeability of particles increases significantly what represents a lot of difficulties during the production of the pharmaceutical composition.

Therefore, the main goal of the present invention is to develop a process of the preparation of a suspension comprising non-micronized ezetimibe micro-particles having small particle size and high specific surface area, wherein said ezetimibe micro-particles are prepared without micronization process, and wherein said process prevents secondary agglomeration of said ezetimibe micro-particles in the suspension. The process according to the present invention provides the product with the required bioavailability.

Micronization of ezetimibe substance leads to an increase of triboelectric effects that tremendously worsen bulk properties of powder substance during the process of the preparation of the pharmaceutical composition and additionally increases the secondary agglomeration during the process of the preparation of the pharmaceutical composition and during the storage of ezetimibe substance.

The average particle size and specific surface area of the non-micronized ezetimibe micro-particles present in the suspension obtained by the process according to the present invention are within values that provide the required bioavailable ezetimibe product. Average particle size of ezetimibe micro-particles can be measured on-line during the preparation of the suspension by laser diffraction measurement or by recovering ezetimibe micro-particles from the suspension. Specific surface area of ezetimibe micro-particles can be measured on the ezetimibe powder recovered from the de-agglomerated and homogenized suspension.

The term 'small particle size' refers to non-micronized ezetimibe micro-particles present in the suspension having an average primary particle size of less than 30 µm, preferably less than 20 µm, more preferably less than 10 µm and most preferably less than 5 µm. The particles preferably have a size or more than 0.5 µm and more preferably of more than 1 µm. The term 'primary particle' refers to crystallites or single crystal particles obtained during the crystallization process, that can be stuck together in agglomerates or can be suspended as free particles in suspension.

Size distribution of ezetimibe particles was determined by laser diffraction by using Malvern Mastersizer 2000 laser diffraction instrument. Samples for analysis were prepared by dispersing a weighed amount of ezetimibe in a few ml of dispersion medium (50 ml of purified water with surfactant).

The term 'high specific surface area' refers to non-micronized ezetimibe micro-particles present in the suspension having specific area of at least 2.5 m²/g, preferably at least 3 m²/g. More preferably the surface area is between 2.5 m²/g and 10 m²/g and most preferably between 3 and 10 m²/g.

Specific surface area was measured on TriStar 3000 instrument using 6 point BET analysis with nitrogen as the adsorbent gas.

The term 'wet homogenizer' refers to any known laboratory and/or industry scale device. Preferred wet homogenizers are high pressure homogenizers, such as homogenizers produced by APV, wet pearl grinders, such as Hosokawa Alpine agitated ball mill, ultra sound processors, such as Hielscher ultrasonic processor, and in particular high shear mixers and colloid mills, such as IKA Turrax® and colloid mills produced by Fryma.

Non-micronized ezetimibe particles are particles which are obtained by de-agglomeration and homogenization of an ezetimibe suspension by a wet-homogenizer, preferably a high pressure homogenizer and in particular a high shear mixer or a colloid mill.

Micronization is the process of reducing the average diameter of a solid material's particles. Usually, the term micronization is used when the particles that are produced are only a few micrometres in diameter. However, applications in the pharmaceutical's industry often require average particle diameters of the nanometer scale. Traditional micronization techniques are based on friction to reduce particle size. Such methods include milling and grinding, crushing, cutting, impact grinding and jet milling. These methods result in the formation of dry powders and not of homogeneous suspensions.

According to the present invention a process for preparing a suspension of ezetimibe particles is provided comprising the steps of
(i) suspending ezetimibe particles in a solvent and
(ii)de-agglomerating and homogenizing the suspension with a wet-homogenizer.

### Step (i) is conducted by

(i-a) dissolving ezetimibe in a solvent,
(i-b) adding the solution of step (i-a) to an anti-solvent,
(i-c) recovering the obtained precipitate from the resulting suspension,
(i-d) drying the precipitate,
(i-e) suspending the dried precipitate in a solvent, preferably in water.

The main goal of the invention can be achieved by any of the following ways:
(A) preparation of agglomerated ezetimibe micro-particles by crystallization process in order to prepare agglomerates which exhibit excellent bulk properties that are of vital importance in the process of the preparation of the pharmaceutical composition and which can easily be de-agglomerated and homogenized in the suspension by a wet homogenizer, preferably a high shear mixer or colloid mill, or
(B) preparation of ezetimibe form S (ezetimibe tert-butanol solvate), as defined and prepared according to the process described in our co-pending patent application EP 07 001 537.5, that can be easily de-solvated, de-agglomerated and homogenized in the suspension by a wet homogenizer, preferably a high shear mixer or colloid mill,
(C)preparation of needle shaped particles of ezetimibe having an average size of more than 30 µm, preferably of 30 µm to 100 µm which can easily be homogenized in the suspension by a wet homogenizer to obtain a suspension comprising non-micronized ezetimibe micro-particles having small particle size and high specific surface area.

All above disclosed processes enable to obtain a suspension comprising non-micronized ezetimibe micro-particles having small particle size and high specific surface area in a solvent, preferably in water, with improved bioavailability of ezetimibe in the final pharmaceutical composition. In such way efficiency and simplicity of the process for the preparation of the pharmaceutical composition comprising ezetimibe as an active substance is achieved and the obtained pharmaceutical composition has the required bioavailability.

The first embodiment of the present invention is the process of the preparation of a suspension comprising non-micronized ezetimibe micro-particles having small particle size and high specific surface area comprising the steps of:
(i') preparation of agglomerated ezetimibe micro-particles,
(ii') de-agglomeration and homogenization in a suspension by high shear mixer or colloid mill.

According to the first embodiment step (i) is conducted by
(i-a) dissolving ezetimibe in a solvent,
(i-b) adding the solution of step (i-a) to an anti-solvent,
(i-c) recovering the obtained precipitate from the resulting suspension,
(i-d) drying the precipitate,
(i-e) suspending the dried precipitate in a solvent, preferably in water.

Preferably, the process comprising the steps of:
(i-a) dissolving ezetimibe in a solvent selected from the group consisting of lover alcohols, amines, ketons or any mixtures thereof, preferably methanol, ethanol and isopropanol or any mixtures thereof,
(i-b) addition of the obtained solution into an anti-solvent selected from the group consisting of toluene, cyclic or linear C₅ to C₆ hydrocarbons, water or any mixtures thereof, preferably acidified water,
(i-c) recovering the obtained precipitate from suspension,
(i-d) drying the precipitate.

More preferably, the first embodiment of the present invention is the process of the preparation of a suspension comprising non-micronized ezetimibe micro-particles having small particle size and high specific surface area by the preparation of agglomerated ezetimibe micro-particles comprising the steps of:
(i-a) dissolving ezetimibe prepared according to any known process and being in any known polymorphic form or any mixture thereof, as for example anhydro form A, hydrated form H or form S, in a solvent selected from the group consisting of lover alcohols, amines, ketons or any mixtures thereof, preferably methanol, ethanol and isopropanol or any mixtures thereof, by heating solution from 20 °C to reflux temperature,
(i-b) adding the obtained solution into an anti-solvent selected from the group consisting of toluene, cyclic or linear C₅ to C₆ hydrocarbons, water or any mixtures thereof, preferably acidified water, wherein the temperature of anti-solvent is maintained at between 1°C and reflux temperature, preferably between 5°C and reflux temperature, more preferably between 1°C and 55°C or between 20°C and 80 °C, even more preferably between 45 °C and 55 °C, in a period of time from 5 minutes to 10 hours, in discrete portions or by continuous flow,
(i-c) recovering the obtained precipitate from suspension,
(i-d) drying the precipitate,
wherein the obtained agglomerated ezetimibe micro-particles are further de-agglomerated and homogenized in a suspension by a wet homogenizer, preferably by a high pressure homogenizer and in particular a high shear mixer or colloid mill.

When water is used as anti-solvent in step b), the pH is preferably controlled between 2 and 7, more preferably between 2 and 4, and even more preferably around 3.

The starting ezetimibe substance can be prepared according to the process as described in our co-pending patent application EP 07 001 537.5.

The obtained agglomerated ezetimibe micro-particles have an average primary particle size between 1 µm to 15 µm, preferably between 1 µm to 10 µm, and the size of the agglomerates can be between 30 µm to 300 µm, preferably between 50 µm to 150 µm.

The obtained agglomerated ezetimibe micro-particles have excellent bulk properties such as for example flowability, low triboelectric chargeability, reduced hygroscopicity.

The agglomerated ezetimibe micro-particles can be easily de-agglomerated and homogenized by the process comprising the following steps:
(i-e) suspending agglomerated ezetimibe micro-particles obtained by the process as described above in a solvent, preferably in water,
(ii) de-agglomerating and homogenizing of the obtained suspension by a wet homogenizer, preferably a high shear mixer or colloid mill, to prepare a suspension of ezetimibe micro-particles having small particle size and high specific surface area.

Concentration of ezetimibe in the solvent during the above steps can range from 0.01 g/mL to 1 g/mL, preferably 0.02 g/mL to 0.2 g/mL, more preferably from 0.04 g/mL to 0.1 g/mL, or from 0.05 g/mL to 0.5 g/mL. Temperature can range from 4°C to 95°C, preferably from 20°C to 70°C, more preferably 20°C to 60°C or 40 °C to 60 °C. Efficiency of de-agglomeration and homogenization step is controlled by measurement of particle size distribution.

High shear mixer or colloid mill can be selected from many known marketed products that are used in laboratory and/or in industry as for example Turrax™.

Optionally, laboratory and/or industry scale ultrasound processor can be used during any of previously described steps, as standalone application or as add-on to high-shear mixer or colloid mill applications.

During any previously described steps pharmaceutically acceptable anionic, cationic and non-ionic surfactants can be optionally added, preferably in a concentration around and above micelle concentration, to control degree of agglomeration of the formed particles.

Optionally, water soluble polymers such as for example polyethylene glycol having molecular weight between 100 and 10,000, povidone with K value in the range 5 to 100, soluble cellulose ether derivatives such as low viscosity grades of hydropropylmethyl cellulose having viscosity of 2% aqueous solution between 0.5 and 20 mPas, hydroxyethyl cellulose, hydroxypropyl cellulose, polyvinyl alcohol, graft copolymer of polyethylene glycol and polyvinyl alcohol or the mixtures thereof can be added to control the degree of agglomeration of the formed particles during any of the previously described steps, in a concentration which can be around or above the critical micelle concentration (CMC).

The second embodiment of the present invention is the process of the preparation of a suspension comprising non-micronized ezetimibe micro-particles having small particle size and high specific surface area from ezetimibe tert-butanol solvate (ezetimibe form S), comprising the following steps:
(i) suspending ezetimibe tert-butanol solvate in a solvent, preferably in water,
(ii) de-agglomerating and homogenizing of the obtained suspension of ezetimibe by a wet homogenizer, preferably a high shear mixer or colloid mill.

Ezetimibe of form S can be prepared according to the process described in European patent application EP 07 001 537.5

Preferred concentration of ezetimibe in the solvent can range from 0.01 g/mL to 1 g/mL, preferably 0.02 g/mL to 0.2 g/mL, more preferably from 0.04 g/mL to 0.1 g/mL. Temperature can range from 4 °C to 95 °C, preferably 20°C to 60°C, more preferably 40 °C to 60 °C. Efficiency of de-agglomeration and homogenization step is controlled by measurement of particle size distribution.

High shear mixer or colloid mill can be selected from many known marketed products that are used in laboratory and/or in industry as for example Turrax™.

Surprisingly it was found out that even larger particles of ezetimibe form S that possesses good bulk properties of powder substance are easily de-solvated if suspended in solvent, preferably in water. By using a wet homogenizer and in particular a high shear mixer or colloid mill in the de-agglomeration and homogenization step the process of de-solvatation is even faster and more effective.

The third embodiment of the present invention is the process of the preparation of a suspension comprising non-micronized ezetimibe micro-particles having small particle size and high specific surface area from needle shaped particles by a process comprising the following steps:
(i) suspending ezetimibe needle shaped particles in a solvent, preferably in water,
(ii) de-agglomerating and homogenizing of the obtained suspension by a wet homogenizer in order to prepare a suspension of ezetimibe micro-particles having small particle size and high specific surface area.

The ezetimibe needles used as starting material preferably have an average size of 30 µm to 100 µm, more preferably 30 µm to 60 µm. The ezetimibe particles can be prepared by any process. They may be of any polymorphic form, for example anhydro form A, hydrated form H or form S. It is also possible to use a mixture of different polymorphic forms.

The concentration of the ezetimibe needles in the solvent is preferably within a range of from 0.01 g/mL to 1 g/mL, preferably from 0.05g/mL to 0.5 g/mL. The temperature may range from 4 °C to 95 °C, preferably from 20°C to 60°C. Efficiency of de-agglomeration and homogenization step is controlled by measurement of particle size distribution.

During any previously described steps pharmaceutically acceptable anionic, cationic and non-ionic surfactants can be optionally added, preferably in a concentration around and above micelle concentration, to control degree of agglomeration of the formed particles.

Optionally, water soluble polymers such as for example polyethylene glycol having molecular weight between 100 and 10,000, povidone with K value in the range 5 to 100, soluble cellulose ether derivatives such as low viscosity grades of hydropropylmethyl cellulose having viscosity of 2% aqueous solution between 0.5 and 20 mPas, hydroxyethyl cellulose, hydroxypropyl cellulose, polyvinyl alcohol, graft copolymer of polyethylene glycol and polyvinyl alcohol or the mixtures thereof can be added to control the degree of agglomeration of the formed particles during any of the previously described steps, in a concentration which can be around or above the critical micelle concentration (CMC).

From any of the above described de-agglomerated and homogenized suspensions in water prepared by the processes according to the present invention, ezetimibe can be recovered from suspension in order to control efficiency of de-agglomeration and stability of formed particles that are not subjected to problems of severe secondary agglomeration. The aim of optional recovery of ezetimibe micro-particles is to measure particle size distribution and/or specific surface area of ezetimibe micro-particles present in the suspension.

The process of the present invention results in the formation of a suspension comprising non-micronized ezetimibe micro-particles of small particle size and high specific surface area wherein non-micronized ezetimibe micro-particles have primary particle size of less than 30 µm, preferably less than 20 µm, more preferably less than 10 µm and most preferably less than 5 µm and specific surface area of at least 2.5 m²/g, preferably at least 3 m²/g. More preferably the surface area is between 2.5 - m²/g and 10 m²/g and most preferably between 3 and 10 m²/g. The particles preferably have a size or more than 0.5 µm and more preferably of more than 1 µm. Said suspension can be directly used in the process of the preparation of pharmaceutical composition comprising ezetimibe and at least one other pharmaceutical, acceptable ingredient.

Non-micronized ezetimibe micro-particles of small particle size and high specific surface area can be isolated from the suspension prepared according to the present invention.

Pharmaceutical compositions may be prepared by well known technological processes such as direct compression, wet granulation, dry granulation or lyophilization.

The suspension comprising non-micronized ezetimibe micro-particles of small particle size and high specific surface area can be advantageously used directly in the preparation of solid pharmaceutical compositions. Pharmaceutical compositions can preferably be prepared by a process comprising the steps of preparing an ezetimibe suspension according to the present invention, spraying the suspension onto a solid pharmaceutically acceptable excipient or a mixture of solid pharmaceutically acceptable excipients and forming the mixture into a pharmaceutically dosage form. Preferably the pharmaceutically acceptable excipient or the mixture of pharmaceutically acceptable excipients is granulated by a granulation process, preferably a wet granulation process and the ezetimibe suspension is sprayed onto the excipient or mixture of excipients during granulation. One or more excipients can be dissolved in the ezetimibe suspension before spraying the same onto the one or more excipients. The mixture or the granulate of the one or more excipients sprayed with the ezetimibe suspension can then be filled into capsules or compressed into tablets.

Preferably the pharmaceutical compositions are prepared by a process of wet granulation of a homogenous mixture of pharmaceutically acceptable ingredients which are preferably selected from the group consisting of diluents, disintegrants, solubilisers, binders and/or stabilizers by processes and equipments known from the state of the art such as for example high shear granulators and/or fluid bed granulators, wherein the suspension is sprayed onto the powder mixture of excipients containing no active ingredient. The obtained granulate can be filled directly into hard capsules made of e.g. gelatin or hydropropylmethyl cellulose or processed to tablets by mixing the granulate optionally with further excipients selected from the group consisting of diluents, disintegrants, glidants and/or lubricants and compressing it into tablets.

In another embodiment of present invention the binder can be dissolved in the suspension according to the present invention and the obtained dispersion can be sprayed onto the mixture of pharmaceutically acceptable pharmaceutical ingredients selected from the group consisting of diluents, disintegrants, solubilisers, stabilizers by processes and equipments known from the state of the art such as high shear granulators and/or fluid bed granulators. In a special embodiment of the present invention the solvent can be evaporated from the suspension according to the present invention. Further pharmaceutically acceptable ingredients selected from the group consisting of diluents, solubilisers, stabilizers, antitacking agents, can be dissolved or suspended in the suspension before evaporation of solvents. The evaporation of solvents can be performed by processes known from state of the art such as spray drying, evaporation of solvents in a stream of dry gas such as air or nitrogen, or evaporation of solvents under reduced pressure. The obtained dry product can be formulated into solid composition by direct compression or through dry granulation with subsequent steps of milling and sieving the obtained granulate and compression to solid composition such as tablets directly or mixed with further pharmaceutically acceptable excipients selected from diluents, disintegrant, glidants and/or lubricants. The solid composition of the present invention in the form of tablets preferably have the hardness determined by radial hardness tester Erweka in the range 25 to 100 N, preferably 30 to 80 N.

The non-micronized ezetimibe micro-particles obtained according to the present invention can also be formulated into the pharmaceutical composition as described in WO 2007/003365 and in co-pending patent application EP 07 001 537.5.

The pharmaceutical composition comprises non-micronized ezetimibe micro-particles obtained according to the present invention which can optionally be mixed with other active ingredients such as for example HMG-CoA reductase inhibitors and at least one pharmaceutical acceptable excipient.

The pharmaceutical composition can be in any conventional form, preferably an oral dosage form such as a capsule, tablet, pill, liquid, emulsion, granule, suppositories, powder, sachet, suspension, solution, injection preparation and the like. The formulations/compositions can be prepared using conventional pharmaceutically acceptable excipients. Such pharmaceutically available excipients and additives include fillers/diluents, binders, disintegrants, glidants, lubricants, wetting agents, preservatives, stabilizers, antioxidants, flavoring agents, coloring agents, emulsifier. Preferably, the oral dosage form is a tablet.

Suitable diluents include lactose, calcium carbonate, dibasic calcium phosphate anhydrous, dibasic calcium phosphate dehydrate (for example Emcompress^{®}), tribasic calcium phosphate, microcrystalline cellulose (such as for example Avicel^{®} PH 101, Avicel^{®} PH 102 etc), powdered cellulose, silicified microcrystalline cellulose (for example Prosolv^{®}), dextrates (for example Emdex^{®}), dextrose, fructose, glucose, lactitol, lactose anhydrous, lactose monohydrate, spray-dried lactose, magnesium oxide, magnesium carbonate, maltitol, maltodextrin, maltose, mannitol, starch, sucralose, sucrose, xylitol and others. Also, special excipients for direct compression such as cellactose or starlac can be used. Preferably, lactose monohydrate, mannitol and microcrystalline cellulose are used.

Binders are preferably selected from the group consisting of gelatin, guar gum, cellulose derivatives, such as hydroxyethyl cellulose HEC, hydroxyethylmethyl cellulose HEMC, hydroxypropyl cellulose HPC (for example Klucel^{®} EF or Klucel^{®} LF), hydroxypropylmethyl cellulose HPMC (Pharmacoat^{®} 603 or 606), methyl cellulose, polymethacrylates, polyvinyl alcohol, povidone (of different grades, for example povidone K12, K15, K17, K25, K30 etc), starch and its derivatives (hydroxyethyl starch, pregelatinized starch) etc.

Suitable disintegrants include carboxymethyl cellulose sodium, carboxymethyl cellulose calcium, croscarmellose sodium, crospovidone, starch and modified starches (sodium starch glycolate - Primojel®), low substituted hydroxypropyl cellulose, magnesium aluminium silicate, calcium silicate and others.

Suitable surface active agents (solubilising agents or surfactants) include, but are not limited to sodium laurylsulfate, glyceryl esters, polyoxyethylene glycol esters, sucrose esters, polyoxyethylene glycol ethers, polyoxyethylene sorbitan fatty acid esters, sulphate containing surfactants, or polyoxyethylene/polyoxypropylene copolymers. The most preferred is sodium laurylsulfate. The surface active agents preferably have a HLB value in the range of 7 to 40, more preferably 9 to 30.

Possible antioxidants include, but are not limited to, vitamin E acetate, α-tocopherol, ascorbyl palmitate, citric acid, butylated hydroxyanisole (BHA), butylated hydroxytoluene (BHT), propyl gallate, dithiotreitol, or tocopherol polyethyleneglycol succinate (TPGS). Chelating agents can also be used as antioxidants, for example EDTA or cyclodextrins.

Suitable glidants are silicon dioxide, talc and aluminum silicate.

Lubricants are preferably selected from the group consisting of magnesium stearate, sodium stearyl fumarate, sucrose esters of fatty acids, stearic acids and the like.

Sweeteners can be selected from aspartame, saccharin sodium, dipotassium glycirrhizinate, aspartame, thaumatin and the like.

The pharmaceutical compositions are useful in the treatment of different lipid disorders such as primary hypercholesterolemia (heterozygous familial or non-familial) or homozygous sitosterolemia.

### EXAMPLES

### Example 1 (comparative): Preparation of ezetimibe particles by slow addition of neutral water

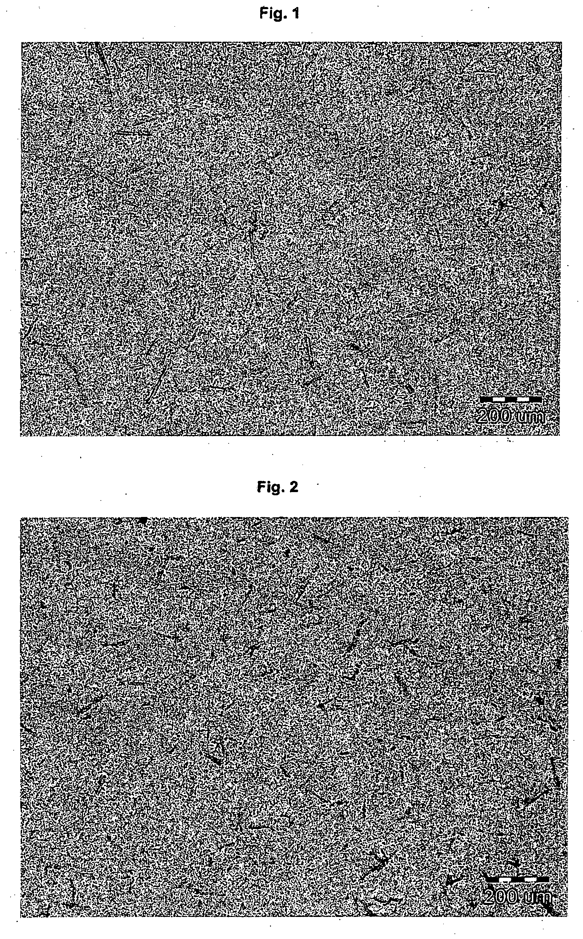

Ezetimibe (2 g) was dissolved in isopropanol (10 mL) at 50 °C, then 5.5 mL of neutral water was quickly added. Further 14.5 mL of water was drop wise added during 3 hours at 50 °C. When addition was completed, the obtained suspension was additionally stirred with magnetic stirrer for 1 hour at 50 °C, then product was filtered and washed with water. Product was dried in vacuum dryer at 50 °C for 10 hours. Yield: 95%, HPLC area of hydrolysis impurities rose from limit of detection to 6.56 %, average particle size was 20 µm, morphology of ezetimibe particles is shown in Figure 1 wherein the attrition of crystals by magnetic stirrer is evident.

The example 1 shows that the hydrolysis impurities in ezetimibe substance are too high for the use in the pharmaceutical composition.

### Example 2 (comparative): Preparation of ezetimibe particles by fast addition of neutral water

Ezetimibe (2 g) was dissolved in isopropanol (13 mL) at 50 °C, then 26 mL of neutral water was added drop-wise in about 5 minutes. The obtained suspension was stirred by magnetic stirrer for additional 2 hours at 50 °C, product was filtered and washed with water. Product was dried in vacuum dryer at 50 °C for 10 hours. Yield: 92%, HPLC area of hydrolysis impurities raised from limit of detection to 1 %, average particle size 29 µm, morphology of ezetimibe particles is shown in Figure 2.

The example 2 shows that the hydrolysis impurities in ezetimibe substance are too high for the use in the pharmaceutical composition.

### Example 3: Preparation of ezetimibe particles by slow addition of acidified water:

Ezetimibe (2 g) was dissolved in isopropanol (10 mL) at 60 °C, then 5.5 mL of acidified water (by HCl_{aq}, pH 2) was quickly added. Further 4.5 mL of acidified water (by HCl_{aq}, pH 2) was dropwise added in 3 hours at 60 °C. When addition was completed, the obtained suspension was additionally stirred with overhead stirrer for 1 hour at 50 °C, then product was filtered and washed with water. Product was dried in vacuum dryer at 50 °C for 10 hours. Yield: 91 %, HPLC area of hydrolysis impurity is below limit of detection.

The example 3 shows that hydrolysis of ezetimibe during the crystallization process is suppressed when using acidified water, however the average particle size is un-appropriate for the direct use in the pharmaceutical composition.

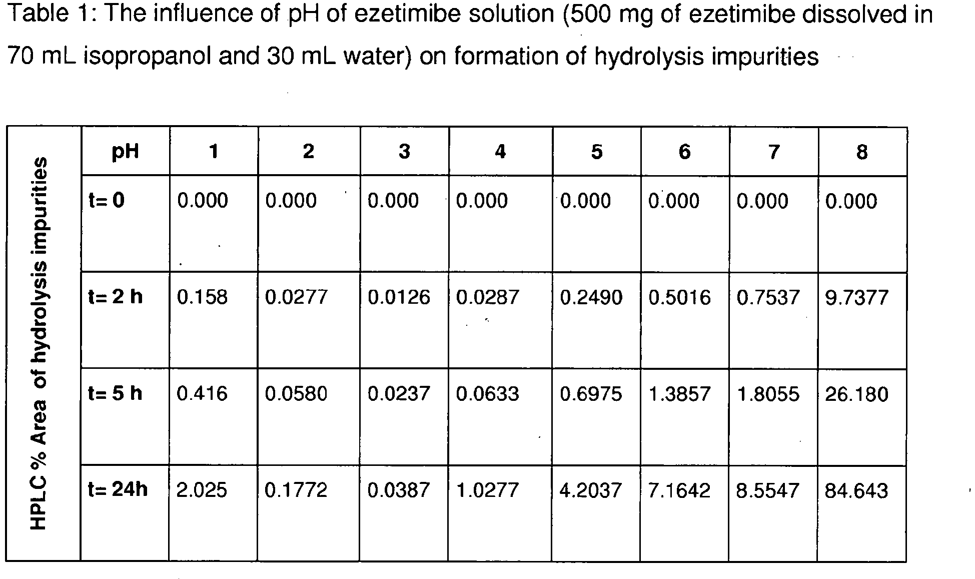

### Example 4a: Preparation of agglomerated ezetimibe micro-particles by addition of ezetimibe solution into acidified water

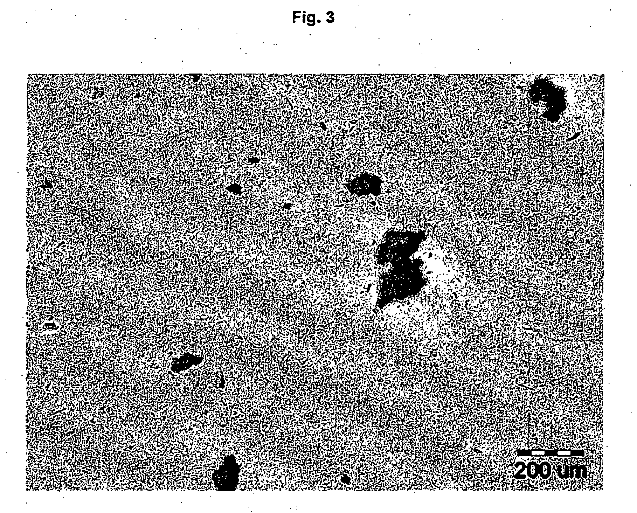

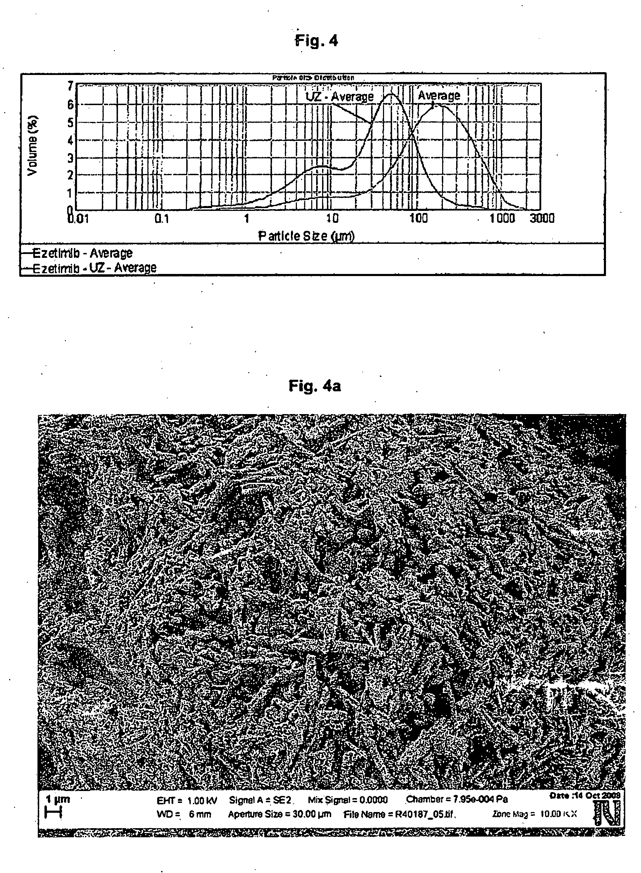

Ezetimibe (2 g) was dissolved in isopropanol (10 mL) at 50 °C. The prepared solution was drop-wise added to 30 mL of acidified water (HCl_{aq}, pH 3.5) in 2 hours during rigorous stirring with overhead stirrer. The obtained suspension was further stirred for 1 hour, filtered and washed with 5 mL of water. Product was dried in vacuum dryer at 50 °C for 10 hours. Yield: 96 %, HPLC area of hydrolysis impurities below limit of detection, morphology of agglomerated ezetimibe micro-particles is shown in figure 3, indicating presence of primary crystalline needle like particles of approximate average primary particle size of about 7 µm, merged in agglomerates of approximate size of about 110 µm as shown in bimodal particle size distribution after de-agglomeration step by ultrasound (UZ) (Figure 4).

### Example 4b: Preparation of agglomerated ezetimibe micro-particles by addition of ezetimibe solution into acidified water

Ezetimibe (300 g) was dissolved in isopropanol (1500 mL) at 50 °C. The prepared solution was filtered and added to 4500 mL of acidified water (HCl_{aq}, pH 3) at 6 °C in 3 hours during rigorous stirring. The obtained suspension was further stirred for 1 hour, filtered and washed with 500 mL of water. Product was dried in vacuum dryer at 50 °C for 10 hours. Yield: 96 %, HPLC purity 99.6 %, morphology of agglomerated ezetimibe micro-particles is shown in figure 4a, indicating presence of primary crystalline needle like particles of approximate average primary particle size below 3 µm, merged in agglomerates of approximate size of about 30 µm.

### Example 5a: Preparation of suspension comprising non-micronized ezetimibe micro-particles: De-agglomeration and homogenization of agglomerated product from example 4a by high shear mixer

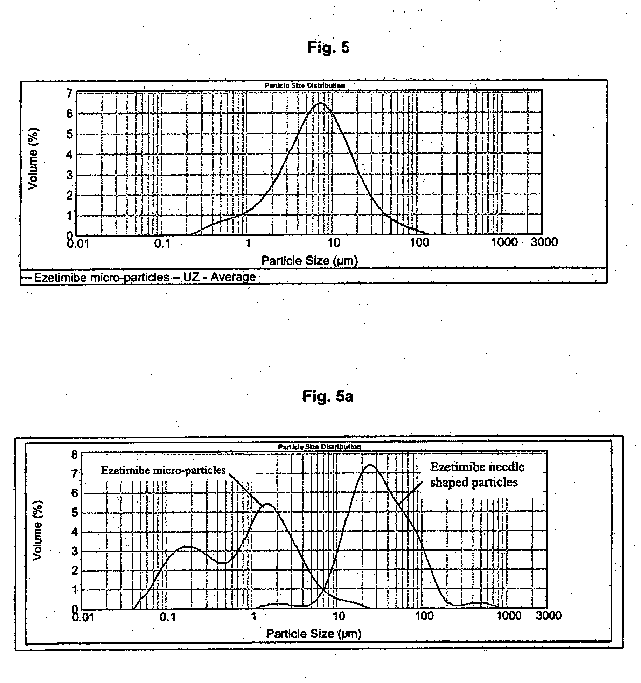

Agglomerated ezetimibe micro-particles (1 g) prepared in example 4a were re-suspended in 25 mL of water at 25 °C. High shear mixer Turrax™ is put in action for 10 minutes with rotation speed of rotor 18000 rpm. Figure 5 shows particle size distribution (measured directly on prepared suspension) of the obtained de-agglomerated and homogenized suspension comprising ezetimibe micro-particles having small particle size and high specific surface area, confirming considerable de-agglomeration of agglomerated particles.

### Example 5b: Preparation of suspension comprising non-micronized ezetimibe micro-particles: De-agglomeration and homogenization of agglomerated product from example 4B by high pressure homogenizer

20 g of povidone is dissolved in 600 g of water. 100 g of agglomerated ezetimibe micro-particles prepared in example 4B is suspended in water solution of povidone. So prepared suspension is passed trough APV homogenizator at a pressure 1000 bar to homogenize suspension (one pass). Particle size distribution (measured directly on prepared suspension) of the obtained de-agglomerated and homogenized suspension comprising ezetimibe micro-particles having small particle size and high specific surface area, confirms complete de-agglomeration of agglomerated particles in homogenized suspension *comprising non-micronized ezetimibe micro-particles.*

### Example 5c: Preparation of suspension comprising non-micronized ezetimibe micro-particles: De-agglomeration and homogenization of needle shaped product prepared according to example 3 by wet pearl grinder

20 g of povidone is dissolved in 600 g of water. 100 g of ezetimibe needle shaped particles prepared according to example 3 is suspended in water solution of povidone. So prepared suspension is passed trough wet pearl grinder at 1200 RPM several times to get stabile suspension. Figure 5a shows particle size distribution (measured directly on prepared suspension) of the obtained homogenized suspension comprising ezetimibe micro-particles having small particle size and high specific surface area, confirming considerable de-agglomeration of agglomerated particles.

### Example 6: Preparation of agglomerated ezetimibe micro particles by addition of ezetimibe solution into acidified water

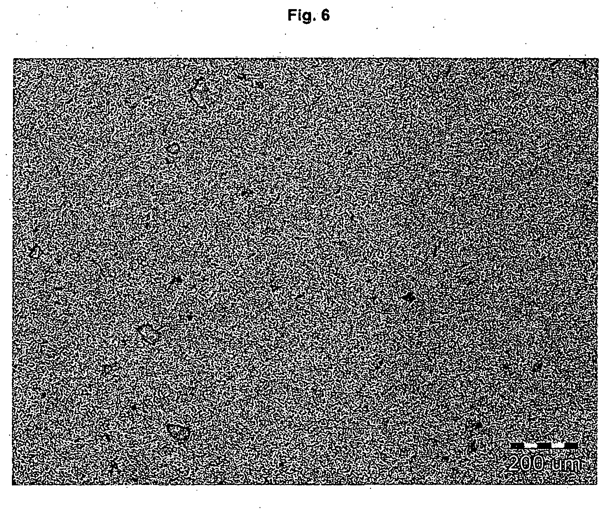

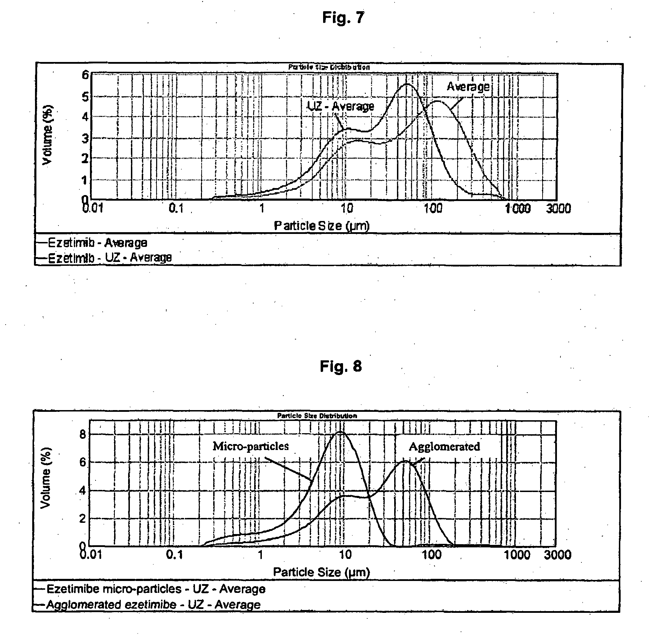

Ezetimibe (100 g) was dissolved in isopropanol (500 mL) at 50 °C. The obtained solution was drop-wise added to 1500 mL of acidified water (HCl_{aq}, pH 3) in 2 hours during rigorous stirring with overhead stirrer. The obtained suspension was further stirred for 1 hour, filtered and washed with 5 mL of water. Product was dried in vacuum dryer at 50 °C for 10 hours. Yield: 96 %, HPLC area of hydrolysis impurities below limit of detection, morphology of particles is shown in Figure 6, indicating presence of primary crystalline needle like particles of approximate average size about 9 µm merged in agglomerates of approximate average size of about 100 µm as shown in bimodal particle size distribution after de-agglomeration step by ultrasound (UZ) (Figure 7).

### Example 7: Preparation of suspension comprising ezetimibe micro-particles: De-agglomeration and homogenization of agglomerated product from example 6 by high shear mixer and characterization of particles after recovering from suspension

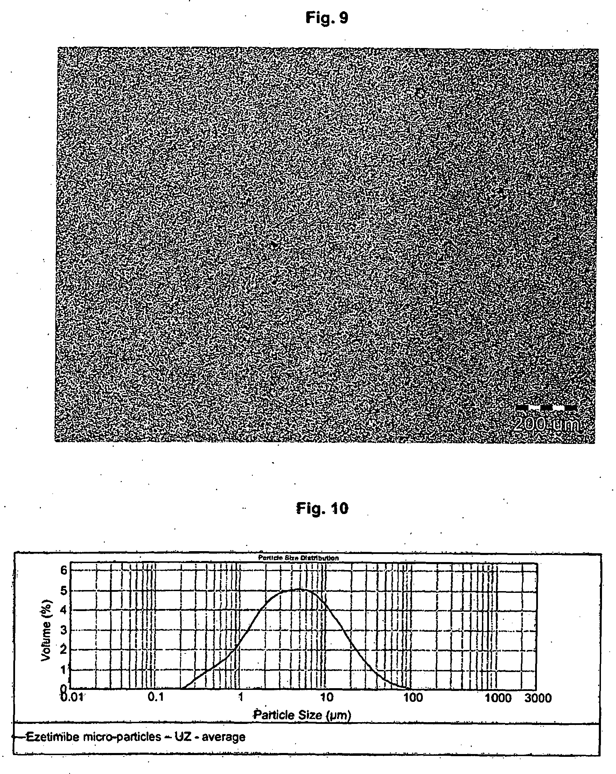

Ezetimibe (5 g) as obtained in example 7 was re-suspended in 50 mL of water. High shear mixer IKA Turrax™ is put in action for 30 minutes with rotation speed of rotor 24000 rpm. Temperature of homogenized suspension was maintained around 40 °C. De-agglomerated and homogenized ezetimibe was recovered from suspension by filtration. Figure 8 shows particle size distribution of the obtained de-agglomerated and homogenized suspension comprising ezetimibe micro-particles having small particle size and high specific surface area confirming considerable de-agglomeration of merged particles. Figure 9 shows morphology of product, showing needle-shaped primary particles. Specific surface area measured on recovered material is 2,7 m²/g.

### Example 8: Preparation of suspension comprising ezetimibe micro-particles: De-solvatation and homogenization of ezetimibe form S by high shear mixer and characterization of particles after recovering from suspension:

Anhydrous ezetimibe (30 g) was dissolved in tert-butanol (204 mL) by heating suspension to 60 °C and magnetic stirring, until clear solution was obtained. Solution was allowed to cool to 33 °C, when seeding crystals of ezetimibe form S were added. Crystallization started, suspension was allowed to cool to 28 °C and product was left to crystallize at this temperature for 18 hours. Dense suspension was recovered by filtration and product was dried in vacuum dryer at 40 °C. Yield: 34 g of ezetimibe form S.

The obtained ezetimibe form S (5 g) was re-suspended in 50 mL of water. High shear mixer Turrax™ is put in action for 30 minutes with rotation speed of rotor 24000 rpm. Temperature of homogenized suspension was maintained at around 40 °C. De-agglomerated and homogenized ezetimibe was recovered from suspension by filtration and dryed in vacuum dryer at 50 °C for 10 hours. Anhydrous ezetimibe was obtained. Average particle size of recovered de-agglomerated ezetimibe micro-particles is less than 8 µm as shown with particle size distribution in figure 10. Specific surface area measured on recovered material is 6 m²/g.

### Examples 9 to 40: Preparation of ezetimibe tablets

Pharmaceutical preparations were prepared having the composition set out in the following tables.

For the preparation of the pharmaceutical compositions solubilizing agent (as for example sodium lauryl sulfate) and/or binder (as for example povidone) were added to a suspension comprising non-micronized ezetimibe. The obtained granulation dispersion was then sprayed onto the powder mixture comprising diluent (as for example lactose monohydrate, mannitol, microcrystalline cellulose), disintegrant (as for example Ac-di-Sol, crospovidone, primojel, L-HPC) and optionally any other additives in a granulation machine. Either fluid bed granulator or high shear mixer was used.

The optional second active ingredient was added intragranulary or extragranulary, into the suspension comprising ezetimibe or it was granulated separately and added to the compression mixture comprising ezetimibe.

Lubricant (as for example magnesium stearate) was admixed and the obtained mixture was pressed into tablets using appropriate compression tool. Alternatively, only part of disintegrant was added intragranularly and the rest extragranularly.

### Examples 9-28

The compositions for the subsequent Ezetimibe 10 mg tablets were as follows:

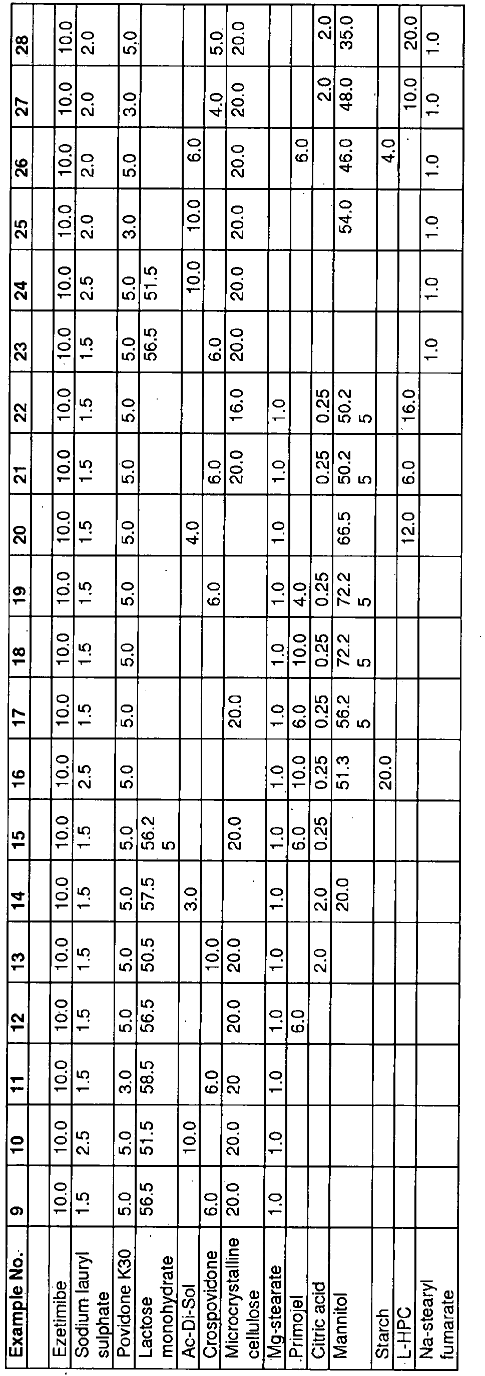

### Examples 29-40

The compositions for the Ezetimibe 10 mg tablets comprising additional active ingredient were as follows:

| **Example No.** | **29** | **30** | **31** | **32** | **33** | **34** | **35** | **36** | **37** | **38** | **39** | **40** |
|---|---|---|---|---|---|---|---|---|---|---|---|---|
| | | | | | | | | | | | | |
| Ezetimibe | 10.0 | 10.0 | 10.0 | 10.0 | 10.0 | 10.0 | 10.0 | 10.0 | 10.0 | 10.0 | 10.0 | 10.0 |
| Atorvastatin calcium | | | | | | | 21.69 | | | 21.69 | | |
| Rosuvastatin calcium | | | | | 20.8 | | | | | | | 20.8 |
| Simvastatin | 40.0 | 40.0 | | 40.0 | | 40.0 | | 40.0 | 40.0 | | 40.0 | |
| Fenofibrate | | | 50.0 | | | | | | | | | |
| Sodium lauryl sulphate | 2.0 | 2.0 | 2.0 | 2.0 | 2.0 | 2.0 | 2.0 | 2.0 | 2.0 | 2.0 | 2.0 | 2.0 |
| Povidone K30 | 3.0 | 3.0 | 3.0 | 3.0 | 3.0 | 3.0 | 3.0 | 3.0 | 3.0 | 3.0 | 3.0 | 3.0 |
| Lactose monohydrate | | | 209.0 | 209.0 | | | | 213.0 | | | 209.0 | 282.2 |
| Ac-Di-Sol | | 10.0 | 10.0 | 10.0 | 10.0 | 10.0 | | | 10.0 | | 10.0 | 10.0 |
| Crospovidone | | | | | | | 6.0 | 6.0 | | 6.0 | | |
| Microcrystalline cellulose | 110.0 | 114.0 | 120.0 | 110.0 | 123.2 | 114.0 | 236.31 | 120.0 | 224.0 | 246.31 | 50.0 | 50.0 |
| Na-stearyl fumarate | 4.0 | 4.0 | 4.0 | 4.0 | 4.0 | 4.0 | 4.0 | 4.0 | 4.0 | 4.0 | 4.0 | 4.0 |
| Primojel | 4.0 | | | | | | | | | | | |
| Citric acid | | | | | 2.0 | 2.0 | | | | | | |
| Ascorbic acid | 2.0 | 2.0 | 2.0 | 2.0 | | | 2.0 | 2.0 | 2.0 | 2.0 | | |
| Mannitol | 215.0 | 215.0 | | | 225.0 | 215.0 | 115.0 | | 105.0 | 105.0 | 54.0 | |
| HPMC | | | | | | | | | | | 8.0 | 8.0 |
| L-HPC | 10.0 | | | | | | | | | | 10 | 10 |

### Example 41: Synthesis of Ezetimibe

### 1st step: 3-{(2S,3R)-2-[4-(benzyloxy)phenyl]-1-(4-fluorophenyl)-4-oxoazetidin-3-yl}-N-methoxy-N-methylpropanamide

Toluene (458 ml), N,O-dimethylhydroxylamine hydrochloride (27 g, 0.277 mol, 1.5 equiv.), and methyl 3-{(2S,3R)-2-[4-(benzyloxy)phenyl]-1-(4-fluorophenyl)-4-oxoazetidin-3-yl}propanoate (80.0 g, 0.184 mol) were charged into a dry flask under inert atmosphere. The mixture was cooled to -10 ± 3 °C with stirring, followed by addition of 12 % diethylaluminum chloride solution (DEAC) in toluene (568 g, 0.565 mol) over 1.5 h. Starting material was consumed within 1 - 2 h after complete addition of DEAC solution. The excess of reagent was then destroyed by careful addition of 20 % NH₄Cl (233 g, 0.87 mol) over 1.5 h at the temperature of 5 ± 5 °C. The layers were separated, the organic phase was washed with 1M NaOH solution (400 ml) and filtered. The clear filtrate was concentrated to give crude amide as a viscous oil (74.6 g) of about 95 % chromatographic purity.

### 2nd step: (3R,4S)-4-[4-(benzyloxy)phenyl]-1-(4-fluorophenyl)-3-[3-(4-fluorophenyl)-3-oxopropyl]azetidin-2-one

A 15 % solution of (4-fluorophenyl)magnesium bromide (814.5 g, 0.613 mol) was charged into a dry flask under inert atmosphere and cooled to -12 ± 3 °C with stirring. To the resulting suspension a solution of the crude product of the previous step (74.6 g) in dry tetrahydrofuran (260 ml) was added over 1 h at the same temperature interval. After about 2.5 h the starting amide was practically consumed. The reaction was quenched by careful addition of 20 % NH₄Cl (800 ml) over 1.5 h at the temperature of -12 ± 3 °C. The mixture was warmed to room temperature and then extracted with ethyl acetate (2 x 600 ml). The organic phase was washed with 7 - 8 % NaHCO₃ solution (1210 ml), filtered and concentrated to give a crude product (74.48 g), which contained 68.7 wt% of the title ketone (51.17 g, 56.0 %).

### 3rd step: (3R,4S)-4-[4-(benzyloxy)phenyl]-1-(4-fluorophenyl)-3-[(S)-3-(4-fluorophenyl)-3-hydroxypropyl]azetidin-2-one (O-Benzylezetimibe)

Asymmetric ruthenium catalyst solution was first prepared as follows. Dichloro(mesitylene)ruthenium(II) dimer (30.6 mg, 0.0523 mmol) and N-[(1S,2S)-2-amino-1,2-diphenylethyl]piperidine-1-sulfonamide (45.3 mg, 0.126 mmol) were suspended in acetonitrile (5.3 ml) at room temperature. The mixture was heated at 80 °C for 3 - 3.5 h under nitrogen atmosphere. The resulting turbid orange-red solution of the catalyst was cooled to room temperature and kept under nitrogen until use.

To a solution of (3R,4S)-4-[4-(benzyloxy)phenyl]-1-(4-fluorophenyl)-3-[3-(4-fluorophenyl)-3-oxopropyl]azetidin-2-one (4.34 g, 8.72 mmol) in acetonitrile (16.7 ml) and water (87 µl), heated at 40 ± 2 °C, formic acid/triethylamine (5/2 molar ratio) reagent (5.486 g) and catalyst solution were added in eight portions in 6 h intervals. After the conversion to O-benzylezetimibe is judged to be complete or, eventually, brought to a standstill, ethyl acetate (35 ml) and water (35 ml) were added to the mixture and stirred. The upper organic phase was washed with sat. NaCl solution and concentrated. Toluene (35 ml) was added to the residue and the mixture concentrated again. The residue was purified by chromatography on silica gel (52 g) with toluene/ethyl acetate (first 30:1, later 5:1) analyzing the fractions by TLC. Fractions containing product spot as a major component were combined and concentrated to give crude product (4.14 g) of about 90 % chromatographic purity. This was crystallized by dissolving it in boiling ethyl acetate (6 ml), followed by slow addition of diisopropylether (18 ml). After stirring at room temperature for 2 h, the crystalline product was collected by filtration, washed with a mixture of ethyl acetate (3 ml) and diisopropylether (9 ml) and dried. The product (2.929 g) contained O-benzylezetimibe and its epimer in a 93:7 ratio (both together of about 99.2 % chromatographic purity), therefore, the yield of pure O-benzylezetimibe was 2.697 g (61.9 %).

### 4th step: (3R,4S)-1-(4-fluorophenyl)-3-[(S)-3-(4-fluorophenyl)-3-hydroxypropyl]-4-(4-hydroxyphenyl)azetidin-2-one (Ezetimibe)

O-benzylezetimibe (24.00 g, 93% pure, 44.23 mmol), tetrahydrofuran (48 ml), ethanol (72 ml), and 5 % Pd on activated charcoal (0.588 g of water wet powder, containing 48.45 % water and 5.11 % Pd on a dry basis) were charged into a flask under inert atmosphere. After heating to 50 ± 2 °C, the inert atmosphere was replaced by hydrogen and the mixture was hydrogenated with vigorous stirring at low pressure of hydrogen for about 22 h. Debenzylation performed under balloon of hydrogen gave the best results. The mixture was cooled, purged with nitrogen, filtered and washed with ethanol (5 ml). The clear filtrate was concentrated to give crude ezetimibe (22.8 g), from which its epimer and other by-products were removed by several consecutive crystallizations from a mixture of 2-propanol and water, acidified with conc. HCl (pH ≈ 3) in a ratio of 11:9 (v/v). Particular crystallization was carried out by preparing saturated solution of impure material in a boiling mixture (cca 4.3 ml/g), followed by stirring at room temperature for about 3 h. Precipitated material was filtered and washed with fresh solvent (cca. 1.7 ml/g). Altogether, 459 ml of solvent mixture was required for five cycles; it was prepared by mixing of 2-propanol (259 ml) and acidified water (212 ml). In this way 13.94 g of pure ezetimibe was prepared with 99.85 % chromatographic purity and 99.93/0.07 ezetimibe to 3'R-epimer ratio. In order to prepare agglomerated ezetimibe, 13.62 g of this material was dissolved in 2-propanol (68 ml) at 50 °C, followed by addition of hot solution within 3 h into chilled (7 °C) water (204 ml), acidified with conc. HCl to pH = 2 - 3. After stirring at 7 °C is continued for another hour, the precipitate was filtered, washed with water (30 ml) and dried in a vacuum oven. Temperature of drying was increased gradually from 30 to 40 °C in several hours, then the product was ground and drying continued at 60 °C until less than 0.5 % of water was found by KF analysis. In this way anhydro form B of ezetimibe (12.73 g, 70.2 %) was obtained.

## Claims

1. A process for preparing a suspension of ezetimibe particles comprising the steps of
(i) suspending ezetimibe particles in a solvent and
(ii) de-agglomerating and homogenizing the suspension with a wet-homogenizer,
wherein step (i) is conducted by
(i-a) dissolving ezetimibe in a solvent,
(i-b) adding the solution of step (i-a) to an anti-solvent,
(i-c) recovering the obtained precipitate from the resulting suspension,
(i-d) drying the precipitate,
(i-e) suspending the dried precipitate in a solvent, preferably in water.

2. The process of claim 1, wherein the solvent is selected from the group consisting of lower alcohols; amines, ketones or a mixture thereof, preferably methanol, ethanol and isopropanol or a mixture thereof, and wherein the anti-solvent is selected from the group consisting of toluene, cyclic or linear C₅ to C₆ hydrocarbons, water or a mixture thereof, preferably water.

3. The process of any one of claims 1 to 2, wherein in step (ii) the suspension of step (i) is de-agglomerated and homogenized with a high pressure homogenizer, a high shear mixer or a colloid mill.

4. The process of any one of claims 1 to 3, wherein a pharmaceutically acceptable anionic, cationic and non-ionic surfactants and/or a water soluble polymer is added in step (i) or (ii).

5. The process of any one of claims 1 to 4, wherein the ezetimibe particles produced in step (ii) have an average primary particle size of less than 30µm, preferably less than 20 µm, more preferably less than 10 µm and most preferably less than 5 µm.

6. The process of any one of claim 1 to 5, wherein the ezetimibe particles produced in step (ii) have a specific surface area of at least 2.5m²/g, preferably at least 3m²/g.

7. A process for preparing a pharmaceutical composition comprising the steps of preparing an ezetimibe suspension according to any one of claims 1 to 6, spraying the suspension onto a solid pharmaceutically acceptable excipient or a mixture of solid pharmaceutically acceptable excipients and forming the mixture into a pharmaceutically dosage form.

8. The process of claim 7, wherein the pharmaceutically acceptable excipient or the mixture of pharmaceutically acceptable excipients is granulated by a granulation process and the ezetimibe suspension is sprayed onto the excipient or mixture of excipients.

9. The process of claim 7 or 8, wherein one or more excipients are dissolved in the ezetimibe suspension before spraying the suspension onto the one or more excipients.

10. The process of any one of claims 7 to 9, wherein the mixture or the granulate is filled into capsules or compressed into tablets.

## Patentansprüche

1. Verfahren zur Herstellung einer Suspension von Ezetimib-Partikeln, das die folgenden Schritte umfasst:
(i) Suspendieren von Ezetimib-Partikeln in einem Lösungsmittel und
(ii) Deagglomerieren und Homogenisieren der Suspension mit einem Nasshomogenisator, bei dem Schritt (i) durchgeführt wird durch
(i-a) Lösen von Ezetimib in einem Lösungsmittel,
(i-b) Zugeben der Lösung aus Schritt (i-a) zu einem Anti-Lösungsmittel,
(i-c) Gewinnen des erhaltenen Präzipitats aus der resultierenden Suspension,
(i-d) Trocknen des Präzipitats,
(i-e) Suspendieren des getrockneten Präzipitats in einem Lösungsmittel, vorzugsweise in Wasser.

2. Verfahren nach Anspruch 1, bei dem das Lösungsmittel aus der Gruppe ausgewählt wird, die aus niederen Alkoholen, Aminen, Ketonen oder einer Mischung davon besteht, vorzugsweise Methanol, Ethanol und Isopropanol oder eine Mischung davon, und wobei das Anti-Lösungsmittel aus der Gruppe ausgewählt ist, die aus Toluol, cyklischen oder linearen C₅ bis C₆ Kohlenwasserstoffen, Wasser oder einer Mischung davon besteht, vorzugsweise Wasser.

3. Verfahren nach einem der Ansprüche 1 bis 2, bei dem die Suspension aus Schritt (i) in Schritt (ii) mit einem Hochdruckhomogenisator, einem Intensivmischer (high shear mixer) oder einer Kolloidmühle deagglomeriert und homogenisiert wird.

4. Verfahren nach einem der Ansprüche 1 bis 3, bei dem in Schritt (i) oder (ii) ein pharmazeutisch verträgliches anionisches, kationisches oder nicht-ionisches Tensid und/oder ein wasserlösliches Polymer zugegeben wird.

5. Verfahren nach einem der Ansprüche 1 bis 4, bei dem die in Schritt (ii) hergestellten Ezetimib-Partikel eine durchschnittliche Primärteilchengröße von weniger als 30 µm, vorzugsweise weniger als 20 µm, besonders bevorzugt weniger als 10 µm und am meisten bevorzugt weniger als 5 µm haben.

6. Verfahren nach einem der Ansprüche 1 bis 5, bei dem die in Schritt (ii) erhaltenen Ezetimib-Partikel eine spezifische Oberfläche von mindestens 2,5 m²/g, vorzugsweise mindestens 3 m²/g haben.

7. Verfahren zur Herstellung einer pharmazeutischen Zusammensetzung, das die Schritte Herstellen einer Ezetimib-Suspension gemäß einem der Ansprüche 1 bis 6, Sprühen der Suspension auf einen festen pharmazeutisch verträglichen Hilfsstoff oder eine Mischung von festen pharmazeutisch verträglichen Hilfsstoffen und Formen der Mischung zu einer pharmazeutischen Darreichungsform umfasst.

8. Verfahren nach Anspruch 7, bei dem der pharmazeutisch verträgliche Hilfsstoff oder die Mischung von pharmazeutisch verträglichen Hilfsstoffen durch ein Granulierungsverfahren granuliert und die Ezetimib-Suspension auf den Hilfsstoff oder die Mischung von Hilfsstoffen gesprüht wird.

9. Verfahren nach Anspruch 7 oder 8, bei dem vor dem Sprühen der Suspension auf den oder die Hilfsstoffe ein oder mehrere Hilfsstoffe in der Ezetimib-Suspension gelöst werden.

10. Verfahren nach einem der Ansprüche 7 bis 9, bei dem die Mischung oder das Granulat in Kapseln gefüllt oder zu Tabletten gepresst wird.

## Revendications

1. Procédé de préparation d'une suspension de particules d'ézétimibe, comportant les étapes suivantes :
i) mettre des particules d'ézétimibe en suspension dans un solvant,
ii) et soumettre la suspension à désagrégation et homogénéisation, au moyen d'un homogénéisateur opérant en milieu humide, dans lequel on réalise l'étape (i) en effectuant les opérations suivantes :
i-a) dissoudre l'ézétimibe dans un solvant,
i-b) verser la solution issue de l'étape (i-a) dans un non-solvant,
i-c) récupérer, à partir de la suspension résultante, le précipité formé,
i-d) faire sécher ce précipité,
i-e) et mettre le précipité séché en suspension dans un solvant, et de préférence dans de l'eau.

2. Procédé conforme à la revendication 1, dans lequel le solvant est choisi dans l'ensemble formé par les alcools inférieurs, amines et cétones et les mélanges de tels composés, mais de préférence par du méthanol, de l'éthanol, de l'isopropanol et leurs mélanges, et dans lequel le non-solvant est choisi dans l'ensemble formé par du toluène, les hydrocarbures cycliques ou linéaires en C₅ ou C₆, de l'eau et les mélanges de tels composés, mais est de préférence de l'eau.

3. Procédé conforme à l'une des revendications 1 à 2, dans lequel, dans l'étape (ii), on soumet la suspension issue de l'étape (i) à désagrégation et homogénéisation à l'aide d'un homogénéisateur opérant sous haute pression, d'un mélangeur à fort cisaillement, ou d'un broyeur à colloïdes.

4. Procédé conforme à l'une des revendications 1 à 3, dans lequel on ajoute, au cours de l'étape (i) ou (ii), un ou des tensioactif(s) anionique(s), cationique(s) et/ou non-ionique(s), pharmacologiquement admissible(s), et/ou un polymère soluble dans l'eau.

5. Procédé conforme à l'une des revendications 1 à 4, dans lequel les particules d'ézétimibe produites à l'issue de l'étape (ii) présentent une taille moyenne de particules primaires inférieure à 30 µm, de préférence inférieure à 20 µm, mieux encore inférieure à 10 µm, et surtout inférieure à 5 µm.

6. Procédé conforme à l'une des revendications 1 à 5, dans lequel les particules d'ézétimibe produites à l'issue de l'étape (ii) présentent une aire spécifique valant au moins 2,5 m²/g, et de préférence au moins 3 m²/g.

7. Procédé de préparation d'une composition pharmaceutique, comportant les étapes suivantes :
- préparer une suspension d'ézétimibe, conformément à l'une des revendications 1 à 6,
- pulvériser la suspension sur un excipient solide pharmacologiquement admissible, ou sur un mélange d'excipients solides pharmacologiquement admissibles,
- et mettre le mélange obtenu en l'état de forme posologique pharmaceutique.

8. Procédé conforme à la revendication 7, dans lequel on met sous forme de granulés, par une opération de granulation, l'excipient pharmacologiquement admissible ou le mélange d'excipients pharmacologiquement admissibles, et l'on pulvérise la suspension d'ézétimibe sur l'excipient ou le mélange d'excipients.

9. Procédé conforme à la revendication 7 ou 8, dans lequel on dissout un ou plusieurs excipient(s) dans la suspension d'ézétimibe avant de pulvériser la suspension sur le ou les excipient(s).

10. Procédé conforme à l'une des revendications 7 à 9, dans lequel on remplit des capsules avec le mélange ou le granulat, ou l'on met sous forme de comprimés le mélange ou le granulat.
